# EUROPEAN PATENT SPECIFICATION

(11) **EP 2 488 871 B1**
(45) Date of publication and mention of the grant of the patent: **04.01.2017**
(21) Application number: 10774160.5
(22) Date of filing: 18.10.2010
(51) Int. Cl.: G01N 33/543

(54) **AN ASSAY METHOD INVOLVING THE USE OF MAGNETIC PARTICLES**
TESTVERFAHREN UNTER VERWENDUNG MAGNETISCHER PARTIKEL
PROCÉDÉ D'ANALYSE METTANT EN JEU L'UTILISATION DE PARTICULES MAGNÉTIQUES

(30) Priority: 16.10.2009 SE 0950762; 16.10.2009 US 252400 P
(43) Date of publication of application: 22.08.2012
(73) Proprietor: Åmic AB, Uppsala SE - 751 83 (SE)
(72) Inventor: MENDEL-HARTVIG, Ib, S-756 55 Uppsala (SE)
(74) Representative: Bergenstråhle Group AB
(86) International application number: PCT/EP2010/065600
(87) International publication number: WO 2011/045436

(56) References cited:
- WO-A1-98/34114
- WO-A1-03/103835
- WO-A1-2005/118139
- WO-A1-2006/137785
- WO-A1-2007/149042
- WO-A1-2007/149043
- WO-A2-01/71344
- WO-A2-2005/089082
- WO-A2-2007/110779
- US-A- 6 136 549
- US-A1- 2008 108 151

## Description

### Technical Field of the Invention

] The present invention relates to the field of diagnostic assays, and in particular open or at least partially open lateral flow assays where an analyte to be detected is present in a complex biological sample. The invention makes available devices and methods for improving the handling of samples; for example, improving the reaction kinetics and thus improving the sensitivity of the assay, based on the use of magnetic particles as the solid phase carrying capture molecules.

### Background of the Invention

] Today, diagnostic assays are widespread and central for the diagnosis, treatment and management of many diseases. Different types of diagnostic assays have been developed over the years in order to simplify the detection of various analytes in clinical samples such as blood, serum, plasma, urine, saliva, tissue biopsies, stool, sputum, and skin or throat swabs. These assays are frequently expected to give a fast and reliable result, while being easy to use and cheap to manufacture. Understandably, it is difficult to meet all these requirements in one and the same assay. In practice, many assays are limited by their speed. Another important parameter is sensitivity. Recent developments in assay technology have led to increasingly more sensitive tests that allow detection of an analyte in trace quantities, as well the detection of disease indicators in a sample at the earliest time possible.

] The most common type of disposable assay device consists of a zone or area for receiving the sample, a reaction zone, and optionally a transport or incubation zone connecting the receiving and reaction zone, respectively. These assay devices are known as chromatography assay devices or simply referred to as test strips. They employ a porous material defining a path for fluid flow capable of supporting capillary flow, e.g. a filter material.

] The sample-receiving zone frequently consists of a more porous material, capable of absorbing the sample, and, when separation of blood cells is desired, also effective to trap the red blood cells. Examples of such materials are fibrous materials, such as paper, fleece, gel or tissue, comprising e.g., cellulose, wool, glass fiber, asbestos, synthetic fibers, polymers, or mixtures of the same.

] The transport or incubation zone commonly consists of the same or similar materials, often with a porosity other than that in the sample-receiving zone. Likewise, the reaction zone, which may be integrated with the incubation zone, or constituting the most distal part thereof, commonly consists of similar, absorbing fibrous materials, or any of the above listed materials.

] In an assay device or strip test, the porous material(-s) is (are) assembled on a carrier, such as a strip of thermoplastic material, paper, cardboard or the like. Further, a cover can be provided, said cover having at least one aperture for receiving the sample, and an aperture or transparent area for allowing a reading of the result of the assay.

] Nitrocellulose materials are frequently used as the matrix constituting the transport or reaction zone, connecting the receiving zone and the reaction zone. A significant disadvantage with nitrocellulose is its high non-specific binding of proteins and other bio-molecules. Present test strips, however, often handle a surplus of sample, reducing the influence of this binding. It is however desirable to minimize the sample volume, in line with the tendency to miniaturize the entire test, including minimizing the amounts of reagents, without compromising accuracy and reliability.

] A specific type of assay devices is the non-porous assay, for example the open lateral flow device as disclosed in WO 03/103835, WO 2005/089082, WO 2005/118139, and WO 2006/137785.

] WO 03/103835 briefly mentions the incorporation of "processing compartments, elements and/or devices" in an assay device having a capillary structure facilitating or driving the capillary flow of a liquid along said structure. These "processing compartments, elements and/or devices" include "magnetic means for trapping magnetic components of said liquid". Further, WO 2003/103835 teaches that "magnets or means for detection of magnetic substances can also be arranged in or around the flow paths. Thereby, magnetic particles can be trapped and retained at desired locations in the structure, and the magnetic property of the particles can thereby be used as a marker or indicator of successful transport to a certain point in the system. Furthermore, magnetic particles can be coated with substances with biological affinity and used in different kinds of assays." The teaching of WO 2003/103835 is focused on the use of magnetic properties as another marker. While suggesting that magnetic particles could also be used as carriers, for example coated with substances with biological affinity, WO 2003/103835 does not show how this would be put into practice.

] WO 2005/089082 mentions the use of magnetic particles in a non-porous assay, where magnetic means are used for the separation of unwanted components of the sample.

] WO 2006/134546 concerns the use of magnetic labels in a sensing device comprising at least one sensing surface, the sensing surface comprising at least one sort of binding sites capable of specifically attaching to at least one sort of biological entities linked to the magnetic labels. The sensing device further comprises at least one magnetic sensor element, the sensing device further comprising distinction means for distinguishing between magnetic labels specifically attached to the binding sites versus labels non-specifically attached in a manner that is time-resolved.

] WO 2007/110779 discloses an assay method and device, where a sample is mixed with magnetically susceptible particles, and where said particles can be manipulated via an applied magnetic field.

] WO 2007/129275 describes a system where magnetic particles are moved over a sensor surface within a reaction chamber, wherein analyte-specific probes or analyte-analogue are bound to the sensor surface.

] US 2008160630 discloses a device for detecting an analyte in a sample, said device comprising a fluidic network having a sample zone, a cleaning zone, and a detection zone. The analyte interacts with magnetic particles which can be moved within the fluidic network using micro coil arrays or mechanically movable permanent magnets.

] WO 2009/009408 concerns a system and methods for detecting analytes such as pathogenic cells. The methods allow for the direct measurement of analytes, such as pathogenic organisms, without the need for sample preparation and/or PCR. The method involves the use of magnetic beads having an outer surface comprising a plurality of ligands which specifically bind to a target analyte.

] Biosensors relying on the use of magnetic particles tend to build on the traditional sandwich assay where the first capture molecule or capture element is bound to the magnetic bead, the second capture molecule or capture element is bound to the solid phase, and the main focus is on utilizing the magnetic properties for accurate detection, down to single bead sensitivity (Janssen et al., in Biosensors and Bioelectronics, 23 (2008) 833-838). US 6,136,549 discloses a magnetic chromatography method for performing a bioassay comprising the steps:
a) providing a chromatographic medium;
b) providing a magnetic field;
c) providing a reaction mixture suspected of, containing an analyte, a reporter ligand that binds to said analyte, and a quantity of magnetic particles with capture ligands for binding said analyte immobilized thereon suspended therein;
d) contacting said chromatographic medium with said reaction mixture such that said reaction mixture flows laterally across said chromatographic medium;
e) applying said magnetic field at a site upon said chromatographic medium, said magnetic field being so applied such that a majority of said magnetic particles suspended within said reaction mixture is caused to become captured upon said medium at said site where said magnetic field is applied; and
f) analyzing said majority of magnetic particle captured upon said chromatographic medium.

] There is a need for further improved kinetics, increased sensitivity and specificity in methods and devices for biochemical and biomolecular assays, in particular for diagnostic assays where the requirements for sensitivity and accuracy are very high.

### Summary of the Invention

] The present inventors make available a method for the detection of an analyte in a liquid sample using a lateral flow assay device, preferably an open lateral flow assay device, including at least one sample addition zone, at least one reaction zone, and at least one sink on a surface, said zones forming a capillary flow path for said sample on said surface; a first capture molecule, capable of binding to said analyte, and a second capture molecule also capable of binding to said analyte, wherein said first capture molecule carries a first label which is not a magnetic label, and said second capture molecule is attached to a magnetic particle.

] It is disclosed that said magnetic particle preferably has a size in the interval of about 5 nm to about 5000 nm, and more preferably about 50 to about 500 nm. Said second capture molecule and/or said magnetic particle may also carry a second label, distinguishable from said first label.

] It is disclosed the option that said second capture molecule bound to a magnetic particle forming a capture conjugate, is pre-deposited on said assay device, and said second capture molecule bound to a non-magnetic label, forming a detection conjugate, is added before or simultaneously with the addition of said sample.

] Alternatively, said first capture molecule is pre-deposited on said assay device, and said second capture molecule is added after or simultaneously with the addition of said sample.

] Preferably, both said first and second capture molecules are pre-deposited on said assay device.

] According to another embodiment, freely combinable with the first described embodiment, both said first and said second capture molecules are deposited in said sample addition zone, and capable of being transported along said flow path upon addition of a sample to said sample addition zone, and reacting with the analyte thus forming a detection complex consisting of said analyte, first and second capture molecules, and said label(-s) and magnetic particle.

] In the above embodiment, said first and second capture molecules are transported by the sample, through the influence of capillary force, to said reaction zone, forming said detection complex.

] It is disclosed the possibility that a magnetic force is applied to move said second capture molecule with its attached magnetic particle within said reaction zone. In this disclosure, the detection complex is moved within the reaction zone, for example retarded or accelerated, oscillated or moved back and forth, and preferably finally concentrated to a discrete location using magnetic force before the qualitative or quantitative detection of said first and/or second label.

] Said sample can be any biological, environmental or clinical sample, but is preferably a sample taken from a mammal, the sample chosen from blood, serum, plasma, urine, saliva, tissue biopsies, stool, sputum, and swabs, such as swabs from the skin or mucosal surfaces, such as nose, throat, and genital swabs, to mention some non-limiting examples.

] Said first and second capture molecule can be any suitable molecule with affinity for the target analyte to be detected or quantified, but is preferably chosen from the group consisting of antibodies, antibody fragments, aptamers, and nucleic acid sequences, specific for the analyte to be detected.

] Said first and/or second label can be any suitable label, but is preferably a label chosen from chromophores, fluorophores, radioactive labels, and enzymes.

] There is disclosed a method for the detection of an analyte in a liquid sample using a lateral flow assay device including at least one sample addition zone, at least one reaction zone, and at least one sink, said zones forming a capillary flow path for said sample; a first capture molecule, capable of binding to said analyte, and a second capture molecule also capable of binding to said analyte, wherein said first capture molecule carries a first label which is not a magnetic label, and said second capture molecule is attached to a magnetic particle, wherein said sample, and said first and second capture molecules, are transported along the flow path through the influence of capillary force.

] There is also disclosed a method for the detection of an analyte in a liquid sample using a lateral flow assay device including at least one sample addition zone, one reaction zone, and one sink, said zones forming a capillary flow path for said sample; a first capture molecule, capable of binding to said analyte, and a second capture molecule also capable of binding to said analyte, wherein said first capture molecule carries a first label which is not a magnetic label, and said second capture molecule is attached to a magnetic particle, wherein said first and second capture molecules are manipulated through the influence of magnetic force.

] It is disclosed that magnetic force can also be applied from the "open" side of the assay. Magnetic force can thus be applied, not only through the substrate, drawing the magnetic carrier particles towards the substrate, but from above, or from the open side of the capillary structure. Experiments performed by the inventors show that the magnetic particles concentrate under the liquid-air interface, without leaving the liquid phase. This allows qualitative and quantitative detection of the result from above, unencumbered by the substrate; i.e., without having to compensate for the influence of the substrate on signal strength, distortion etc.

] The inventors also disclose an improved assay device for the detection of an analyte in a sample, said device comprising a sample addition zone, optionally a conjugate zone, a reaction zone, and a sink, said zones forming a flow path; a first capture molecule, capable of binding to said analyte, optionally deposited on the device; and a second capture molecule also capable of binding to said analyte, optionally deposited on said device, wherein said first capture molecule carries a first label which is not a magnetic label, and said second capture molecule is attached to a magnetic particle.

] In a disclosed assay device said magnetic particle preferably has a size in the interval of about 5 nm to about 5000 nm, more preferably about 50 to about 500 nm. Said second capture molecule and magnetic particle optionally also carries a second label, distinguishable from said first label.

] It is disclosed the option that said second capture molecule is pre-deposited on said assay device. Alternatively, said first capture molecule is pre-deposited on said assay device. Preferably, both said first and second capture molecules are pre-deposited on said assay device.

] It is disclosed the possibility that both said first and said second capture molecules are deposited in said sample addition zone, and capable of being transported along said flow path upon addition of a sample to said sample addition zone.

] It is disclosed that it is possible that at least said sink consists of projections, substantially perpendicular to the surface of the device, and having a height, width, and reciprocal spacing such, that capillary flow is induced in the flow path.

] Preferably the flow path consists of micro-pillars being projections substantially vertical in relation to a surface, and having a height, diameter and reciprocal spacing capable of creating lateral flow of the sample in said flow path.

] Alternatively, the flow path comprises a nitrocellulose based material.

] There is disclosed the option that the flow path is covered by a lid. Said lid preferably only offers mechanical protection of the flow path, but does not participate in the creation of capillary flow. When a lid is present, it is arranged at a distance from the flow path, said distance exceeding the capillary distance.

] It is disclosed the alternative that the flow path comprises an area without micro-pillars within the reaction zone. Alternatively, or in addition thereto, an area without micro-pillars is provided adjacent to said flow path in fluid connection therewith.

] Further, the flow path preferably comprises at least one discrete area with micro-pillars having a different height, diameter or reciprocal spacing than the micro-pillars in the surrounding flow path.

] It is disclosed the option that, said device comprises magnetic elements. Said magnetic elements are preferably permanent magnets, incorporated into the device. Alternatively, said magnetic elements are elements capable of generating a magnetic field when subjected to an external signal; e.g., the application of an electric current through a conducting element.

] Further, said device preferably comprises a discrete area in connection with the flow path, said area having features improving the reading of the result of the assay.

] In any of the above embodiments, the device is preferably a disposable assay device.

] Further, in any of the above embodiments, said first and second capture molecule can be any suitable molecule with affinity for the target analyte to be detected or quantified, but is preferably chosen from the group consisting of antibodies, antibody fragments, aptamers, and nucleic acid sequences, specific for the analyte to be detected.

] Further, said first and/or second label can be any suitable label, but is preferably a label chosen from chromophores, fluorophores, radioactive labels, and enzymes.

] The inventors also disclose a device for reading (a reader) the result of an assay performed on an assay device (an assay platform), wherein said device comprises means for reading a signal emitted by, or reflected from said detection complex, means for computing the signal and displaying a result, and means capable of manipulating magnetic components present on said assay device.

] It is disclosed that said means capable of manipulating magnetic components present on said assay device are preferably means for activating an element or elements present in the assay device.

] Alternatively, in another disclosure regarding the device, said means capable of manipulating magnetic components present on said assay device are magnetic means capable of being brought within an effective distance from the assay device for manipulating magnetic components present on the assay device. Said magnetic means can be for example permanent magnets, brought in contact with or within an effective distance from the assay device; or electromagnets, present at an effective distance from the device, and activated when the influence of magnetic force is desired.

] In either of the above disclosures, said means capable of manipulating magnetic components present on said assay device may also comprise first means present in said device for reading the result (the reader), which activate second means, present in the assay device (the assay platform), which upon activation generate a magnetic field.

] There is also disclosed a device for reading the result of an assay performed on an assay device, wherein said device comprises a detector capable of reading a signal emitted from or reflected from at least one detection complex present in a defined location of said assay device, and means capable of drawing magnetic components present on said assay device to said location before reading said signal.

] In the above disclosure, said means may comprise first means present in said device for reading the result, which activate second means, present in the assay device, which upon activation generate a magnetic field capable of drawing magnetic components present on said assay device to said defined location before reading said signal.

] In either of the above disclosures, the reading preferably is chosen from the detection and/or quantification of color, fluorescence, radioactivity or enzymatic activity.

] The method, assay device, and reader according to an embodiment of the invention have many advantages, mainly related to the improved reaction kinetics of the immunochemical reactions and the increased sensitivity of the assay.

] Another advantage is that the method can be applied on existing assay platforms, and in particular the open lateral flow platform.

] A further advantage is that the deposition of the second capture molecule, covalently coupled to the magnetic particle, to the sample addition zone is easier than separate and discrete covalent immobilization of the capture molecules to one or several positions in the reaction zone omitting activation of the flow channel surface. Thus, the method and device according to various embodiments of the invention makes it possible to handle capture molecules in an environment as far as possible optimized for said entities, without having to take into account the requirements of accurate positioning and immobilization. Instead, the immobilization is achieved by applying an external magnetic force.

] The capture molecule which is attached to a magnetic particle can be manipulated within the flow path, preferably agitated, oscillated, transported, immobilized and the like, in order to improve mixing and thus reaction kinetics, prolong the contact time between analyte and capture molecules, and focus the detection conjugates before reading the result of the assay.

] Further, not only the capture conjugate, but also the detection complex comprising the first and second capture molecules, the analyte, the magnetic particle and one or more labels, can be manipulated, preferably immobilized or oscillated. In the case the system is satiated, the oscillation of the particles during reading of the signal will improve the reading as the particles otherwise would shadow the labels.

] It is also an advantage to be able to concentrate the detection complex to a specific location, within the flow path, or outside it, before reading the result. This is advantageous for example in situations where the background is high.

] Yet a further advantage is that the surface of the assay device requires only minimal or no chemical modification to accommodate the reaction chemistry, as all chemistry resides on the particles.

] These and other features and advantages will be further discussed in the following Detailed Description, which should be read in conjunction with the accompanying drawings.

### Brief Description of the Drawings

] The invention will be described in closer detail below, in the description and non-limiting examples, and with reference to the attached drawings in which:
Figure 1 schematically shows a lateral flow assay platform according to an exemplary embodiment of the invention;
Figure 2 schematically shows the capture and detection of an analyte using two capture molecules, whereof one capture molecule is coupled to a magnetic particle;
Figures 3a, b, c and d schematically show various embodiments of a lateral flow assay device according to the invention;
Figures 4 a and b shows two embodiments of a lateral flow assay platform where a discrete location is formed, here shown as an appendix to the main flow path, in fluid connection therewith; and
Figure 5 shows the results of an analyte binding assay of fluorophore labeled CRP binding measured as signal (RFU) to various concentrations of anti-CRP antibody linked to magnetic beads retarded by a magnet.

### Detailed Description of the Invention

] Before the invention is disclosed and described in detail, it is to be understood that this invention is not limited to particular devices, compounds, configurations, method steps, substrates, and materials disclosed herein as such devices, compounds, configurations, method steps, substrates, and materials may vary somewhat. It is also to be understood that the terminology employed herein is used for the purpose of describing particular embodiments only and is not intended to be limiting since the scope of the present invention is limited only by the appended claims and equivalents thereof.

] It must be noted that, as used in this specification and the appended claims, the singular forms "a", "an" and "the" include plural referents unless the context clearly dictates otherwise.

] If nothing else is defined, any terms and scientific terminology used herein are intended to have the meanings commonly understood by those of skill in the art to which this invention pertains.

] The term "about" as used in connection with a numerical value throughout the description and the claims denotes an interval of accuracy, familiar and acceptable to a person skilled in the art. Said interval is ± 10 %.

] The term "capture molecule" means a molecule suitably chosen for its affinity to the target biological compound or compounds or its affinity to relevant modifications of said target biological compound or compounds. For example, if the target biological compounds are DNA, the capture molecule can be, but is not limited to, synthetic oligonucleotides, analogues thereof, or specific antibodies. A non-limiting example of a suitable modification of a capture molecule is a biotin substituted target biological compound, in which case the probe may bear avidin functionality.

] The term "label" designates an agent which is detectable with respect to its physical distribution or/and the intensity of the signal it delivers, such as but not limited to luminescent molecules (e.g., fluorescent agents, phosphorescent agents, chemiluminescent agents, bioluminescent agents and the like), colored molecules, molecules producing colors upon reaction, enzymes, radioisotopes, ligands exhibiting specific binding and the like.

] Two labels are "distinguishable" when they can be individually detected and preferably quantified simultaneously, without significantly disturbing, interfering or quenching each other.

] The term "manipulated" is intended to mean exerting an influence on the magnetic particles, and the capture molecules bound thereto, or the entire detection complex, comprising a magnetic particle. "Manipulating" comprises both retarding and accelerating the movement of the particles in relation to their movement in the absence of the magnetic force. This term also encompasses that the particles are moved in one, two or three directions, such as back and forth in relation to the flow of sample, transversally in relation to the flow of sample, or oscillated within the sample layer on the assay device. The term also encompasses that the capture molecules, or the entire detection complex, are moved to different locations on the assay device, e.g., returned to the sample addition zone for prolonged contact with the sample, circulated within the reaction zone for improved kinetics, or concentrated in a specific location for reading the result of the assay etc.

] "Sink" as used throughout the claims and the description denotes an area with the capacity of receiving liquid sample.

] The present inventors make available a method for the detection of an analyte in a liquid sample using a lateral flow assay device including at least one sample addition zone, at least one reaction zone, and at least one sink, said zones forming a capillary flow path for said sample; a first capture molecule, capable of binding to said analyte, and a second capture molecule also capable of binding to said analyte, wherein said first capture molecule carries a first label which is not a magnetic label, and said second capture molecule is attached to a magnetic particle.

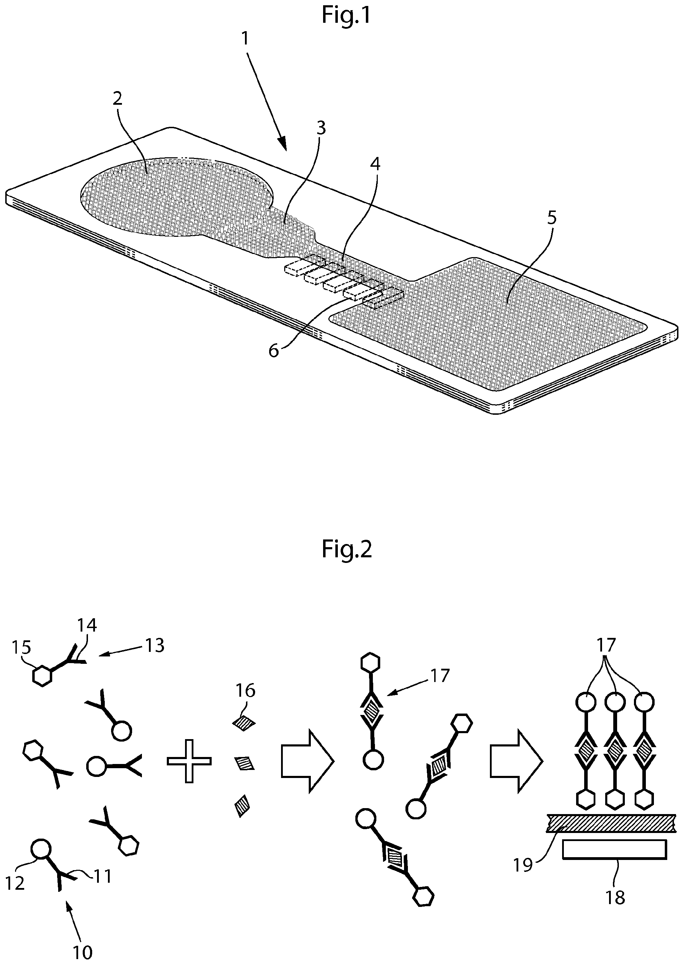

] Figure 2 schematically shows the capture and detection of an analyte 16, using detection conjugates 10 comprising a first capture molecule 11 and a label 12, e.g. a fluorophore, and capture conjugates 13 comprising a second capture molecule 14, which is the same or different from the first capture molecule 11, and a magnetic particle 15. When brought in contact with the analyte 16, the detection and capture conjugates 10 and 13 respectively form a detection complex 17. According to one embodiment of the invention, this detection complex 17 can be manipulated using a magnet 18, e.g. immobilized to a specific location on the surface of an assay device, for example a chip, here schematically indicated as 19.

] In disclosed methods, said magnetic particle preferably has a size in the interval of about 5 nm to about 5000 nm, and more preferably about 50 to about 500 nm.

] Suitable magnetic particles are known from the literature, and available from commercial suppliers; e.g., Chemicell GmbH, of Berlin, Germany; Bioclone Inc., of San Diego, CA, USA; Nanostructured & Amorphous Materials, Inc., of Huston, TX, USA. Known magnetic particles for bioseparation consist of one or more magnetic cores with a coating matrix of polymers, silica or hydroxylapatite with terminal functionalized groups. The magnetic core generally consists either of magnetite (Fe₃O₄) or maghemite (gamma Fe₂O₃) with superparamagnetic or ferromagnetic properties.

] Alternatively, magnetic cores made with magnetic ferrites, such as cobalt ferrite or manganese ferrite can also be produced. Magnetic particles can also be manufactured to order, and given the desired properties for a given application. Non-limiting examples of magnetic particles are given in Table 1:

**Table 1 Examples of magnetic particles**

| Name | Description | Average diameter (µm) | Manufacturer |
|---|---|---|---|
| Uptibeads | polystyrene coated magnetic particles | 0.86 | Uptima, France |
| PN-particles | PNIPAM-coated particles (PN 113 e) | 0.29 | Ademtech, France |
| | PNIPAM-coated particles (PN 145 TTb) | 0.57 | |
| Adembeads | uniform superparamagnetic nanoparticles | 0.2 | |
| IMC AS CR | Alginic acid - magnetite particles | 5-10 | NA |
| AEHMBP | magnetite particles coated with [(2-amino-ethyl)hydroxymethylene]-biphosphonic acid | 0.5 - 1.0 | NA |
| poly(HEMA-co-EDMA)-microspheres | magnetic particles coated with poly(2-hydroxyethyl methacrylate-co-ethylene dimethacrylate) | 2.5 | NA |
| PGMA-microspheres | poly(glycidylmethacrylate) | 2.9 | NA |

] Said second capture molecule and/or said magnetic particle may also carry a second label, distinguishable from said first label.

] It is disclosed the possibility that said first detection conjugate is pre-deposited on said assay device, and said second capture conjugate is added after or together with the addition of said sample. Alternatively, said second capture conjugate is pre-deposited on said assay device, and said first detection conjugate is added after or simultaneously with the addition of said sample. Preferably, both said detection and capture conjugates are pre-deposited on said assay device.

] In the above disclosure, said first and second capture molecules are transported by the sample, through the influence of capillary force, to said reaction zone, forming said detection complex.

] It is disclosed the possibility that a magnetic force is applied to move said second capture molecule with its attached magnetic particle within said reaction zone. The capture conjugate can be retarded, accelerated, oscillated, moved back and forth, or manipulated in any other suitable way. It is disclosed that it is a preferred that, the detection complex is concentrated to a discrete location using magnetic force before the qualitative or quantitative detection of said first and/or second label.

] Said sample is preferably a sample taken from a mammal, chosen from blood, serum, plasma, urine, saliva, tissue biopsies, stool, sputum, and so called swabs, such as swabs from the skin or mucosal membranes, for example but not limited to the nose, throat or genitalia.

] Said first and second capture molecule is preferably chosen from the group consisting of antibodies, antibody fragments, aptamers, and nucleic acid sequences, specific for the analyte to be detected.

] Said first and/or second label is preferably a label chosen from chromophores, fluorophores, radioactive labels, and enzymes. Suitable labels are available from commercial suppliers, providing a wide range of dyes for the labeling of antibodies, proteins, and nucleic acids. There are for example fluorophores spanning practically the entire visible and infrared spectrum. Suitable fluorescent or phosphorescent labels include for instance, but are not limited to, fluoresceins, Cy3, Cy5 and the like. Suitable chemoluminescent labels are for instance but are not limited to luminol, cyalume and the like.

] Similarly, radioactive labels are commercially available, or capture molecules can be synthesized so that they incorporate a radioactive label. Suitable radioactive labels are for instance but are not limited to radioactive iodine and phosphorus; e.g., 125I and 32P.

] Suitable enzymatic labels are for instance but are not limited to horseradish peroxidase, beta-galactosidase, luciferase, alkaline phosphatase and the like.

] Threre is disclosed a method for the detection of an analyte in a liquid sample using a lateral flow assay device including at least one sample addition zone, at least one reaction zone, and at least one sink on a surface, said zones forming a capillary flow path for said sample on said surface; a first capture molecule, capable of binding to said analyte, and a second capture molecule also capable of binding to said analyte, wherein said first capture molecule carries a first label which is not a magnetic label, and said second capture molecule is attached to a magnetic particle, wherein said sample, and said first and second capture molecules, are transported by the sample along the flow path through the influence of capillary force.

] There is also disclosed a method for the detection of an analyte in a liquid sample using a lateral flow assay device including at least one sample addition zone, one reaction zone, and one sink on a surface, said zones forming a capillary flow path for said sample on said surface; a first capture molecule, capable of binding to said analyte, and a second capture molecule also capable of binding to said analyte, wherein said first capture molecule carries a first label which is not a magnetic label, and said second capture molecule is attached to a magnetic particle, wherein said first and second capture molecules are manipulated through the influence of magnetic force. The molecules bound to magnetic particles can be both retarded and accelerated in relation to their movement in the absence of the magnetic force. Retarding the movement of a particle will make it move more slowly than the sample, and can prolong the contact time. Accelerating the particle can help to agitate the sample, also improving contact and kinetics of the reaction. Manipulating the particles also encompasses that the particles are moved in one, two or three directions, such as back and forth in relation to the flow of sample, transversally in relation to the flow of sample, or oscillated within the sample layer on the assay device. This also encompasses that the capture molecules, or the entire detection complex, are moved to different locations on the assay device, e.g. returned to the sample addition zone for prolonged contact with the sample, circulated within the reaction zone for improved kinetics, or concentrated in a specific location for reading the result of the assay, etc.

] According to one embodiment of the invention, the method is applied to a lateral flow assay, for example an assay performed on a platform as schematically shown in Figure 1. This lateral flow assay platform, or so called assay chip 1, has at least one sample zone 2, optionally at least one conjugate zone 3, at least one reaction zone 4 optionally comprising several reaction zones (not shown), placed in parallel between the sample zone and at least one sink 5. The flow path can comprise open or closed paths, grooves and/or capillaries. Preferably the flow path comprises a lateral flow path of adjacent projections, each having a size, shape and mutual spacing such that capillary flow is sustained through said flow path. Figure 1 also schematically indicates the position of one or more magnetic elements 6, either incorporated into the assay platform, or elements of a reader (not shown), which can be brought into operational contact with the assay chip1.

] In one embodiment the flow path is at least partially open. In another embodiment the flow path is entirely open. "Open" in accordance with this invention means that there is no lid or cover at a capillary distance. Thus the lid, if present as a physical protection for the flow path, does not contribute to the capillary flow in said flow path. An open lateral flow path is described for example in the following published applications: WO 2003/103835, WO 2005/089082; WO 2005/118139; WO 2006/137785; and WO 2007/149042. Details on the micro-pillars as well as their height, diameter and reciprocal spacing can be obtained from these documents.

] According to one embodiment of the method, magnetic force is applied to the "open" side of the assay. Magnetic force is thus applied from above, or from the open side of the capillary structure. Experiments performed by the inventors show that the magnetic particles concentrate under the liquid-air interface, without leaving the liquid phase. This allows qualitative and quantitative detection of the result from above, unencumbered by the substrate; i.e., without having to compensate for the influence of the substrate on signal strength, distortion etc.

] The inventors also make available an improved assay device for the detection of an analyte in a sample, said device comprising a sample addition zone, a reaction zone, and a sink, said zones forming a flow path; a first capture molecule, capable of binding to said analyte, optionally deposited on the device; and a second capture molecule also capable of binding to said analyte, optionally deposited on said device, wherein said first capture molecule carries a first label which is not a magnetic label, and said second capture molecule is attached to a magnetic particle.

] In an assay device according to the above disclosure, said magnetic particle preferably has a size in the interval of about 5 nm to about 5000 nm, more preferably about 50 to about 500 nm. Said second capture molecule and magnetic particle optionally also carries a second label, distinguishable from said first label.

] It is disclosed a possibility that said second capture molecule is pre-deposited on said assay device. Alternatively, said first capture molecule is pre-deposited on said assay device. Preferably, both said first and second capture molecules are pre-deposited on said assay device.

] It is disclosed a possibility that both said first and said second capture molecules are deposited in said sample addition zone, and capable of being transported along said flow path upon addition of a sample to said sample addition zone.

] In a preferred embodiment, freely combinable with the above embodiments, at least said sink consists of projections, substantially perpendicular to the surface of the device, and having a height, width, and reciprocal spacing such, that capillary flow is induced in the flow path.

] Preferably the flow path consists of micro-pillars being projections substantially vertical in relation to a surface, and having a height, diameter and reciprocal spacing capable of creating lateral flow of the sample in said flow path.

] As described above, an open lateral flow path has been disclosed and defined for example in the following published applications: WO 2003/103835, WO 2005/089082; WO 2005/118139; WO 2006/137785; and WO 2007/149042.

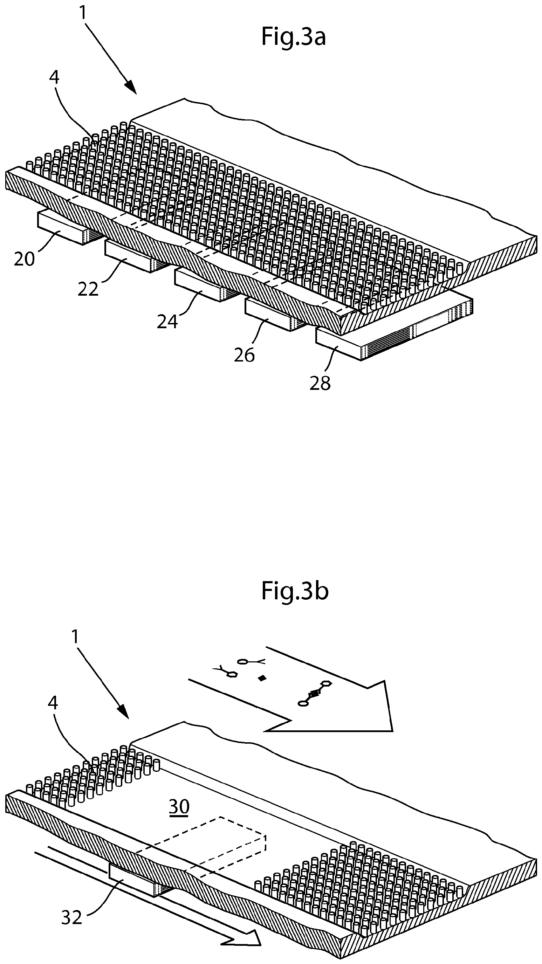

] A device according to the invention is schematically shown in Figures 3a-3d, where a section of the flow path or reaction zone 4 is shown in alternative views, each view illustrating a different embodiment of the invention, freely combinable with other embodiments.

] Fig. 3a shows schematic partial cross-section of an assay device or platform according to one embodiment in which conjugates comprising a capture molecule and a magnetic particle are manipulated using magnetic force, here illustrated as magnetic elements 20, 22, 24, 26, and 28 arranged or brought into operational distance from the assay device to influence the movement and/or position of the capture molecules bound to magnetic particles, or the detection complex, comprising magnetic particles. The magnetic elements 20, 22, 24, 26, and 28 can be brought into operational contact with the device, or activated, serially, in parallel, or in a desired sequence or pattern in order to influence the contact time between capture molecules and the sample, improve mixing and reaction kinetics. This can be achieved, for example, by mechanically moving a magnet, or by activating one or more electromagnets.

] As described above, in relation to the method, also here the molecules bound to magnetic particles herein can also be both retarded and accelerated in relation to their movement in the absence of the magnetic force. Retarding the movement of a particle will make it move more slowly than the sample, and can prolong the contact time. Accelerating the particle can help to agitate the sample, also improving contact and kinetics of the reaction. Manipulating the particles also encompasses that the particles are moved in one, two or three directions, such as back and forth in relation to the flow of sample, transversally in relation to the flow of sample, or oscillated within the sample layer on the assay device. This also encompasses that the capture molecules, or the entire detection complex, are moved to different locations on the assay device; e.g., returned to the sample addition zone for prolonged contact with the sample, circulated within the reaction zone for improved kinetics, or concentrated in a specific location for reading the result of the assay etc.

] Fig. 3b shows an embodiment of an assay device 1 according to another embodiment, having a flow path, here shown as part of the reaction zone 4, in which conjugates comprising a capture molecule and a magnetic particle are aided by a movable magnetic element 32 in bridging a gap or discontinuity 30 in the flow path, e.g. a portion without micropillars, a portion with reduced capillary flow, a time gate, etc.

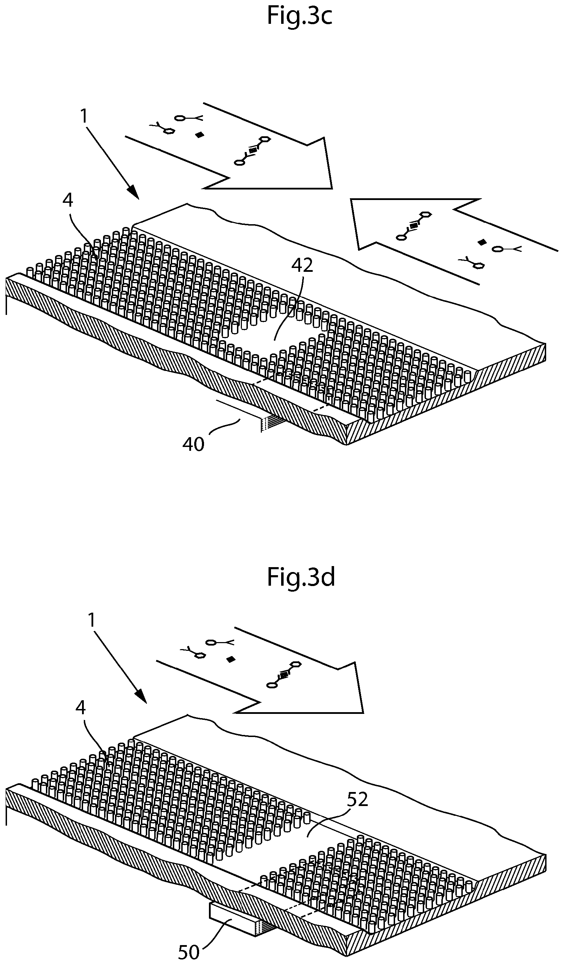

] Fig. 3c shows another embodiment of an assay device 1 according to another embodiment, having a flow path; e.g., the reaction zone 4, where conjugates comprising a capture molecule and a magnetic particle are drawn by magnetic force to one or more specific, discrete locations on the assay platform; e.g., a section of the flow path, a location outside the flow path, or another location, for example in order to facilitate reading the result. A discrete location is here illustrated as an area 42 without micro-pillars within the flow path. An element 40; e.g., a permanent magnet or an electromagnet, is schematically shown in a position corresponding to the location of the area 42. This element can either be integrated in the assay device 1, or preferably part of a device for reading the result.

] Fig. 3d shows another embodiment of an assay device 1 having a flow path, e.g. the reaction zone 4, in which a stationary magnetic element 50 in association with a discontinuity 52 in the flow path, can function as a valve or time gate. The discontinuity in the flow path constitutes a discontinuity in the capillary force, and prevents the mixture of sample and capture molecules from advancing along the flow path under the influence of said capillary force. By activating the magnetic element, the capture molecules bound to magnetic particles, as well as detection complexes comprising magnetic particles, are drawn into the discontinuity, wetting the opposite end of the flow path and restoring capillary flow.

] The assay device according to embodiments of the invention can also be applied to other formats and platforms, such as bibulous materials, e.g. nitrocellulose fiber based materials. An assay device according to embodiments of the invention can also be a reaction vessel, such as a well of a microtiter plate.

] Alternatively, the flow path comprises a nitrocellulose based material.

] In one embodiment, freely combinable with the above embodiments, the flow path is covered by a lid. Said lid preferably does not participate in the creation of capillary flow.

] According to another embodiment, freely combinable with the above embodiments, the flow path comprises an area without micro-pillars within the reaction zone. Alternatively, or in addition thereto, an area without micro-pillars is provided adjacent to said flow path in fluid connection therewith.

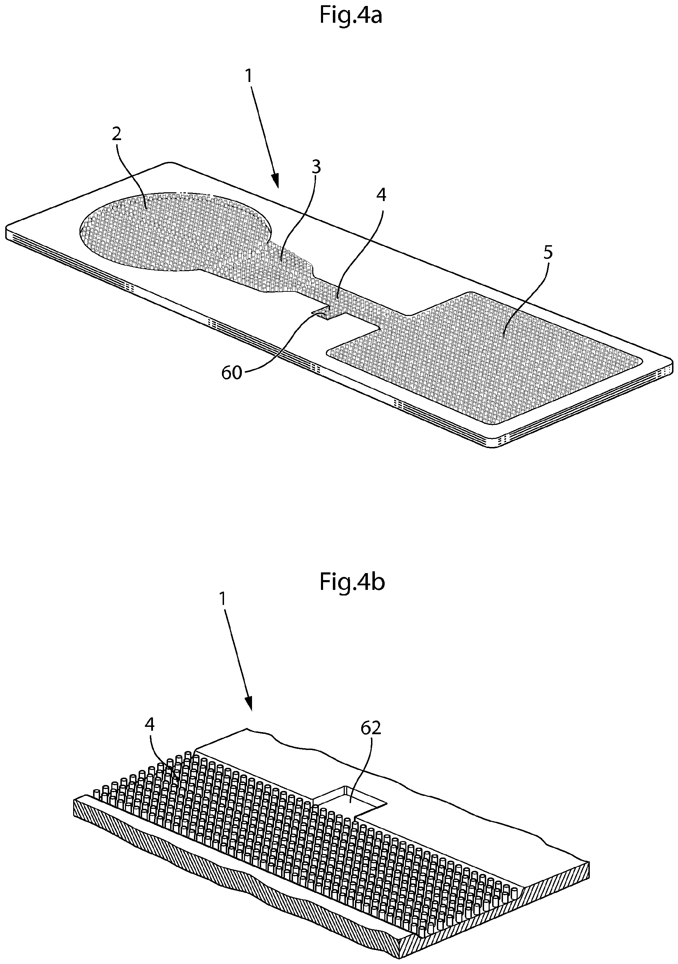

] The preceding is illustrated in Figure 4a, which shows an embodiment of a lateral flow assay platform, or so called assay chip 1, having a sample addition zone 2, a conjugate zone 3, a reaction zone 4, and a sink 5, forming a flow path between said sample addition zone and said sink, where a discrete location 60 is formed, here shown as an appendix to the main flow path, in fluid connection therewith.

] Further, Figure 4b shows how the discrete location, here denoted 62 can be an appendix to the reaction zone 4 on an assay platform or chip 1. This area 62 is also in fluid connection with the flow path, but has however a different structure, here shown as an area without micro-pillars.

] According to another embodiment, freely combinable with the above embodiments, the flow path preferably comprises at least one discrete area with micro-pillars having a different height, diameter or reciprocal spacing than the micro-pillars in the surrounding flow path.

] According to yet another embodiment, also freely combinable with the above embodiments, said device comprises magnetic elements. Said magnetic elements are, for example, permanent magnets, incorporated into the device. Alternatively, said magnetic elements are elements capable of generating a magnetic field when subjected to an external signal; e.g., the application of an electric current through said element.

] Further, said device preferably comprises a discrete area in connection with the flow path, said area having features improving the reading of the result of the assay.

] In any of the above embodiments, the device is preferably a disposable assay device.

] Further, in any of the above embodiments, said first and second capture molecule is preferably chosen from the group consisting of antibodies, antibody fragments, aptamers, and nucleic acid sequences, specific for the analyte to be detected.

] Further, said first and/or second label is preferably a label chosen from chromophores, fluorophores, radioactive labels, and enzymes. Examples of labels are given above in the preceding description, and equally applicable in this context.

] The assay device and methods according to embodiments of the invention can also be applied to other assay formats and assay platforms, such as assays conducted on bibulous materials, such as nitrocellulose fiber based materials. The assay device and methods according to embodiments of the invention can also be applied to assays conducted in suspension, in reaction vessels, or at least partially bound to the walls of reaction vessels.

] The inventors also make available a device for reading (a reader) the result of an assay performed on an assay device (an assay platform), wherein said device comprises means for reading a signal emitted by, or reflected from said detection complex, means for computing the signal and displaying a result, and means capable of manipulating magnetic components present on said assay device.

] According to one embodiment of the device, said means capable of manipulating magnetic components present on said assay device comprise one or more permanent magnets that can be brought in effective contact with or electromagnets activated within effective distance of the assay device or chip.

] In another embodiment of the device, said means capable of manipulating magnetic components present on said assay device are means for inducing a magnetic force, activating electromagnets or other elements present in the assay device.

] Examples of elements that can be activated include, but are not limited to, thermally activated magnetic elements, elements such as coils that are electrically activated, conducting elements that induce a magnetic field when subjected to an electric current, and Fe-based elements that are magnetized when in contact with an external magnet.

] Alternatively, in another embodiment of the device, said means capable of manipulating magnetic components present on said assay device are magnetic means which are part of a device for reading the results of the assay, and capable of being brought within an effective distance from the assay device for manipulating magnetic components present on the assay device. Preferably, electromagnets are arranged in the reader at an effective distance to the assay device, or chip, when in position in the reader, and said electromagnets can then be activated when desired, in order to exert an influence on the magnetic particles in the capture conjugate or in the detection complex.

] In either of the above embodiments, said means capable of manipulating magnetic components present on said assay device may also comprise first means present in said device for reading the result (the reader), which activate second means, present in the assay device (the assay platform), which upon activation generate a magnetic field.

] Another embodiment is a device for reading the result of an assay performed on an assay device, wherein said device comprises a detector capable of reading a signal emitted from or reflected from at least one detection complex present in a defined location of said assay device, and means capable of drawing magnetic components present on said assay device to said location before reading said signal.

] In the above embodiment, said means are first means present in said device for reading the result, which activate second means, present in the assay device, which upon activation generate a magnetic field capable of drawing magnetic components present on said assay device to said location before reading said signal. In either of the above embodiments, the reading preferably is chosen from the detection and/or quantification of color, fluorescence, radioactivity or enzymatic activity.

] It is to be understood that this invention is not limited to the particular embodiments shown here. The following examples are provided for illustrative purposes and are not intended to limit the scope of the invention since the scope of the present invention is limited only by the appended claims and equivalents thereof.

### Examples

### Example 1

] The advantage of using magnetic beads as the solid phase in a lateral flow assay was demonstrated in an analyte binding assay using a standard assay design for comparison i.e. where the capture antibody is bound to the flow path. The analyte used was C-reactive protein (CRP), a marker for inflammation and heart failure. The test was carried out on a non-porous carrier, having a micropillar array defining an open lateral flow path, as described for example in one of WO 2003/103835, WO 2005/089082; WO 2005/118139; WO 2006/137785; and WO 2007/149042. Plastic substrate chips made of Zeonor® (Zeon Corporation, Japan) were manufactured by Åmic AB, Uppsala, Sweden. The chips or assay devices were designed substantially as illustrated in Fig. 1.

] An anti-CRP antibody M701189 (Fitzgerald, USA) was linked to paramagnetic beads of diameter 0,2 µm (Adembeads, Ademtech, France) in reaction buffer (25 mM MES buffer, pH 6.0) forming the product bead-aCRP. Carboxylated beads (4x10E11 particles) were incubated with 50 mg/ml 1-Ethyl-3-(3-dimethylaminopropyl) carbodiimide hydrochloride and 50 mg/ml Sulfo-N-hydroxyl succinimide ester for 30 min at room temperature, in a total volume of 100 µl reaction buffer.

] The beads were washed two times with 100 µl buffer, applying a strong magnet for removal of solutions. Activated beads were subsequently incubated with 132 µl 2.1 mg/ml anti-CRP antibodies for 30 minutes at room temperature. Excess antibody was removed by washing two times with 300 µl reaction buffer followed by two times 300 µl storage buffer (0.1M TrisHCl pH7.5, 0.35 M NaCl, 5mM CaCl2, 0.4% BSA, 0.2% Triton X-100, 0.05% NaN3) and finally resuspended in storage buffer. Fluorophore labeled CRP (CRP*) was obtained by applying Dylight 649 antibody labeling kit (prod nr 53050, Thermo Scientific).

] An analyte binding assay with respect to CRP was run in open lateral flow chips similar to the design shown in Figure 1. The chip was placed in a chip holder with magnets (a stack of three 1mm thick 3x3mm block magnets, Neodym35, ELFA, Sweden) fixed in close proximity to the bottom surface of the chip, below the flow path. The chip holder was then placed in a humidity chamber. 5 µl of storage buffer was added before application of sample. Bead-antiCRP antibody as well as CRP* were diluted separately in serum (TSH free serum, US Biological), and a set of bead-antiCRP antibody dilutions were prepared. Each individual assay sample was prepared by mixing equal volumes of 20 ng/ml CRP* and bead-antiCRP antibody allowing 30 seconds of binding reaction before application of 10 µl mixture in the chip sample zone. Surplus reagents were removed by washing with three times 7.5 µl of serum. As control, the background signal generated by the beads themselves was recorded; i.e., no CRP* was added but bead-antiCRP antibody dilution was applied as sample. In the standard assay protocol, anti-CRP antibody containing chips were produced by deposition of 60 nl of 1.0 mg/ml antibody (in 50 mM Sodium phosphate buffer pH 7.5, 2% Trealose) by Biodot AD3200. The signal intensities were recorded in a prototype line-illuminating fluorescence scanner (Åmic AB, Sweden). The experiment was repeated several times, using different chip variants, and materials prepared and the experiments performed by different persons.

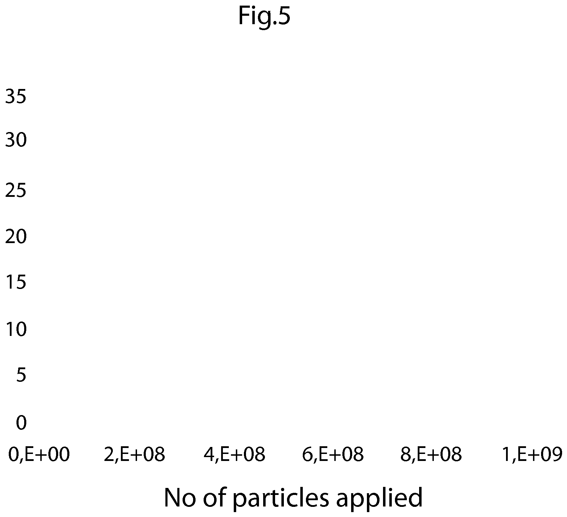

] Fig. 5 shows the results of an analyte binding assay of fluorophore labeled CRP binding to various concentrations of anti-CRP antibody linked to magnetic beads retarded by magnet. Empty symbols display beads mixed with CRP* and filled symbols indicate beads only. Thirty seconds of binding was allowed before application on the chip. The dashed line corresponds to binding of labeled CRP to anti-CRP antibody deposited in the chip path. Different symbols (squares, circles, triangles) in Fig. 5 indicate different runs, performed at the same concentration, but under different conditions (preparation, experimentalist and chip variant).

] It is evident from Fig. 5 that the present invention most significantly (more than 3 times) increases the specific signal in a lateral flow assay design using magnetic beads as solid phase compared to the standard assay having the capture antibodies on the surface of the flow path.

### Example 2

] Experiments were performed with the same or similar chip design as in Example 1, using the same anti-CRP antibody and paramagnetic beads. Contrary to the procedure in Example 1, the magnets (a stack of three 1mm thick 3x3mm block magnets, Neodym35, ELFA, Sweden) were now held above the flow path, in close proximity to the chip. Surprisingly the beads did not leave the liquid phase, but instead accumulated just below the liquid surface. Without wishing to be bound by theory, the inventors speculate that the surface tension was sufficient to prevent the beads from leaving the liquid phase. The experiments showed that magnetic particles can be used in an open lateral flow system, and freely manipulated within the liquid phase, e.g. moved in any direction or even oscillated within the liquid phase.

] In addition to improved mixing and reaction kinetics, this embodiment makes it possible to concentrate the detection conjugates at the liquid-air-interface. This makes it possible to read the signal directly, from the side of the flow path, and not from below, through the substrate as frequently is the case. This in turn obviates problems with signal distortion, signal weakening, and other consequences of measuring through the substrate.

] Further, extracting the detection conjugates from the flow path into the surface of the liquid phase can help to remove the conjugates from the physical structure of the flow path, regardless of this comprising fibers or microstructures, and draw the detection conjugates into a well defined, uniform and discrete location.

] Those skilled in the art will appreciate that the invention and embodiments thereof described herein are susceptible to variations and modifications other than those specifically described. It is to be understood that the invention includes all such variations and modifications. The invention also includes all of the steps and features referred to in this specification, individually or collectively, and any and all combinations of any two or more of said steps or features.

## Claims

1. A method for the detection of an analyte in a liquid sample using an open lateral flow assay device (1) including at least one sample addition zone (2), at least one reaction zone (4), and at least one sink (5) on a substrate, said zones forming a flow path for said sample on said substrate; a first capture molecule on said assay device, capable of binding to said analyte; and a second capture molecule also capable of binding to said analyte, wherein said first capture molecule carries a first label which is not a magnetic label, thereby forming a detection conjugate, and said second capture molecule is attached to a magnetic particle, thereby forming a capture conjugate, wherein the flow path includes micro-pillars being projections substantially vertical in relation to a surface, and having a height, diameter and reciprocal spacing capable of creating capillary lateral flow of the sample in said flow path, and further wherein a magnetic force is applied to the open side of the flow path, concentrating the detection conjugate to the liquid/air interface.

2. The method according to claim 1, including the step of pre-depositing said second capture molecule attached to said magnetic particle on said assay device, and adding said first capture molecule after or simultaneously with the addition of said sample.

3. The method according to claim 1, including the step of pre-depositing both said first and second capture molecules on said assay device.

4. The method according to claim 1, including the step of depositing both said first and said second capture molecules in said sample addition zone wherein each of said capture molecules are transported along said flow path upon addition of a sample to said sample addition zone, and reacting with the analyte thus forming a detection complex consisting of said analyte, first and second capture molecules, and said label(-s) and magnetic particle.

5. The method according to claim 1, wherein said second capture molecule and/or said magnetic particle also carry a second label, distinguishable from said first label.

6. The method according to claim 1, wherein said sample is a sample taken from a mammal, chosen from blood, serum, plasma, urine, saliva, tissue biopsies, stool, sputum, and skin or throat swabs.

7. The method according to claim 1, wherein said first and second capture molecule is chosen from the group consisting of antibodies, antibody fragments, aptamers, and nucleic acid sequences, specific for the analyte to be detected.

8. The method according to claim 1, wherein said first and/or second label is a label chosen from chromophores, fluorophores, radioactive labels, and enzymes.

## Patentansprüche

1. Verfahren zur Detektion eines Analyts in einer flüssigen Probe unter Verwendung einer offenen Lateral-Flow-Assay-Vorrichtung (1), die mindestens eine Probenhinzufügungszone (2), mindestens eine Umsetzungszone (4) und mindestens eine Senke (5) auf einem Substrat beinhaltet, wobei die Zonen eine Strombahn für die Probe auf dem Substrat bilden; ein erstes Fängermolekül auf der Assayvorrichtung, das in der Lage ist, an das Analyt zu binden; und ein zweites Fängermolekül, das ebenfalls in der Lage ist, an das Analyt zu binden, wobei das erste Fängermolekül eine erste Markierung trägt, die keine magnetische Markierung ist, wodurch ein Detektionskonjugat gebildet wird, und das zweite Fängermolekül an dem ein magnetisches Teilchen befestigt ist, damit ein Fängerkonjugat gebildet wird, wobei der Durchflussweg Mikrosäulen beinhaltet, die Vorsprünge sind, die im Wesentlichen vertikal bezüglich einer Oberfläche sind und eine Höhe, einen Durchmesser und eine gegenseitige Beabstandung aufweisen, die in der Lage ist, einen lateralen kapillaren Fluss der Probe in der Strombahn zu erzeugen, und wobei ferner eine magnetische Kraft auf der offenen Seite der Strombahn angewendet wird, die das Detektionskonjugat auf die Flüssigkeit-Luft-Schnittstelle konzentriert.

2. Verfahren nach Anspruch 1, beinhaltend den Schritt des Vorabscheidens des zweiten Fängermoleküls, das an dem magnetischen Teilchen befestigt ist, auf der Assayvorrichtung, und Hinzufügen des ersten Fängermoleküls nach oder gleichzeitig mit dem Hinzufügen der Probe.

3. Verfahren nach Anspruch 1, beinhaltend den Schritt des Vorabschiedens sowohl des ersten als auch des zweiten Fängermoleküls auf der Assayvorrichtung.

4. Verfahren nach Anspruch 1, beinhaltend den Schritt des Abscheidens sowohl der ersten und der zweiten Fängermoleküle in der Probenhinzufügungszone, wobei jedes der Fängermoleküle entlang der Strombahn transportiert wird, wenn eine Probe zur Probenhinzufügungszone hinzugefügt wird, und Umsetzen mit dem Analyt, wodurch ein Detektionskomplex gebildet wird, der aus einem Analyt, ersten und zweiten Fängermolekülen und Markierung(en) und magnetischem Teilchen besteht.

5. Verfahren nach Anspruch 1, wobei das zweite Fängermolekül und/oder das magnetische Teilchen auch eine zweite Markierung trägt, die von der ersten Markierung unterscheidbar ist.

6. Verfahren nach Anspruch 1, wobei die Probe eine Probe ist, die einem Säugetier entnommen wurde, ausgewählt aus Blut, Serum, Plasma, Urin, Speichel, Gewebebiopsien, Stuhl, Sputum, und Haut oder Halsabstrichen.

7. Verfahren nach Anspruch 1, wobei das erste und zweite Fängermolekül ausgewählt ist aus der Gruppe, bestehend aus Antikörpern, Antikörperfragmenten, Aptameren und Nukleinsäuresequenzen, die für das zu detektierende Analyt spezifisch sind.

8. Verfahren nach Anspruch 1, wobei die erste und/oder die zweite Markierung eine Markierung ist, die ausgewählt ist aus Chromophoren, Fluorophoren, radioaktiven Markierungen und Enzymen.

## Revendications

1. Procédé pour la détection d'un analyte dans un échantillon de liquide en utilisant un dispositif d'analyse à flux latéral ouvert (1) incluant au moins une zone d'ajout d'échantillon (2), au moins une zone de réaction (4), et au moins une dépression en surface (5) sur un substrat, lesdites zones formant un chemin de flux pour ledit échantillon sur ledit substrat ; une première molécule de capture sur ledit dispositif d'analyse, susceptible de se lier audit analyte ; et une seconde molécule de capture également susceptible de se lier audit analyte, dans lequel ladite première molécule de capture porte un premier marqueur qui n'est pas un marqueur magnétique, formant de ce fait un conjugué de détection, et ladite seconde molécule de capture est attachée à une particule magnétique, formant de ce fait un conjugué de capture, dans lequel le chemin de flux inclut des micro-piliers qui sont des projections sensiblement verticales par rapport à une surface, et ayant une hauteur, un diamètre et un espacement réciproque susceptibles de créer un flux latéral capillaire de l'échantillon dans ledit chemin de flux, et en outre dans lequel une force magnétique est appliquée au côté ouvert du chemin de flux, concentrant le conjugué de détection sur interface liquide / air.

2. Procédé selon la revendication 1, incluant l'étape de dépôt préalable de ladite seconde molécule de capture attachée à ladite particule magnétique sur ledit dispositif d'analyse, et d'ajout de ladite première molécule de capture après ou simultanément avec l'ajout dudit échantillon.

3. Procédé selon la revendication 1, incluant l'étape de dépôt préalable des deux dites première et seconde molécules de capture sur ledit dispositif d'analyse.

4. Procédé selon la revendication 1, incluant l'étape de dépôt des deux dites première et seconde molécules de capture dans ladite zone d'ajout d'échantillon dans laquelle chacune desdites molécules de capture est transportée le long dudit chemin de flux lors de l'ajout d'un échantillon à ladite zone d'ajout d'échantillon, et de réaction avec l'analyte formant ainsi un complexe de détection constitué par ledit analyte, les première et seconde molécules de capture, et lesdits marqueur(s) et particule magnétique.

5. Procédé selon la revendication 1, dans lequel ladite seconde molécule de capture et/ou ladite particule magnétique portent également un second marqueur, pouvant être distingué dudit premier marqueur.

6. Procédé selon la revendication 1, dans lequel ledit échantillon est un échantillon pris auprès d'un mammifère, choisi parmi le sang, sérum, plasma, urine, salive, biopsies de tissu, selles, crachat et des frottis de gorge ou de peau.

7. Procédé selon la revendication 1, dans lequel lesdites première et seconde molécules de capture sont choisies à partir du groupe constitué par des anticorps, des fragments d'anticorps, des aptamères et des séquences d'acides nucléiques, spécifiques pour l'analyte à détecter.

8. Procédé selon la revendication 1, dans lequel ledit premier et/ou second marqueur est un marqueur choisi à partir de chromophores, fluorophores, marqueurs radioactifs, et enzymes.
